Europäisches Patentamt

European Patent Office

Office européen des brevets

(19)

(11) Veröffentlichungsnummer: **0 192 173**
**B1**

(12) **EUROPÄISCHE PATENTSCHRIFT**

(45) Veröffentlichungstag der Patentschrift: **13.06.90**

(51) Int. Cl.⁵: **A 61 K 9/20**

(21) Anmeldenummer: **86101741.6**

(22) Anmeldetag: **12.02.86**

(54) **Direkttablettierhilfsmittel.**

(30) Priorität: **16.02.85 DE 3505433**

(43) Veröffentlichungstag der Anmeldung:
**27.08.86 Patentblatt 86/35**

(45) Bekanntmachung des Hinweises auf die
Patenterteilung:
**13.06.90 Patentblatt 90/24**

(84) Benannte Vertragsstaaten:
**BE CH DE FR GB IT LI NL**

(56) Entgegenhaltungen:
DE-A-3 012 136
**JOURNAL OF PHARMACEUTICAL SCIENCES,
Band 62, Nr. 1, Januar 1973, Seiten 43-49; S.S.
KORNBLUM et al.: "A new tablet disintegrating
agent: cross-linked polyvinylpyrrolidone"**

Die Akte enthält technische Angaben, die nach
dem Eingang der Anmeldung eingereicht
wurden und die nicht in dieser Patentschrift
enthalten sind.

(73) Patentinhaber: **BASF Aktiengesellschaft
Carl-Bosch-Strasse 38
D-6700 Ludwigshafen (DE)**

(72) Erfinder: **Lang, Siegfried, Dr.
Thomas-Mann-Strasse 22
D-6700 Ludwigshafen (DE)**

(56) Entgegenhaltungen:

**PHARMACEUTISCH WEEKBLAD SCIENTIFIC
EDITION, Band 5, 1983, Seiten 165-171; H.V.
VAN KAMP et al.: "Improvement by super
disintegrants of the properties of tablets
containing lactose, prepared by wet
granulation"**

**R. VOIGT: "Lehrbuch der pharmazeutischen
Technologie", 1975, Seite 188, VEB Verlag Volk
und Gesundheit, Berlin, DD**

**PHARMACOPOEA DANICA, Band III, Ausgabe
IX, 1948, Seite 82, Nyt Nordisk Forlag Arnold
Busck, Kopenhagen, DK**

EP 0 192 173 B1

**Beschreibung**

Die Erfindung betrifft ein Direkttablettierhilfsmittel auf Basis von pulverförmiger Lactose in inniger Vermischung mit einem Bindemittel, vorzugsweise Polyvinylpyrrolidon, und einem Tablettensprengmittel, vorzugsweise vernetztem unlöslichem Polyvinylpyrrolidon.

Die Verwendung von zahlreichen Trägermaterialien, wie mikrokristalliner Cellulose, Cellulose, Dicalciumphosphat, Sorbit und insbesondere Lactose, ist in der pharmazeutischen Industrie bei der Tablettenherstellung weithin üblich.

Die verwendeten Trägermaterialien sollen zahlreiche zum Teil gegenläufige Anforderungen erfüllen, wie gute Fließfähigkeit, gute Komprimierbarkeit bei geringem Druck, hohe Härte und Abreibsfestigkeit und ferner gute Zerfallsneigung nach Einnahme.

Diese Anforderungen werden von handelsüblichen Tablettierhilfsmitteln nur mehr oder weniger gut erfüllt. Deshalb arbeitet man bei der Tablettenherstellung weitere Hilfsstoffe ein, wie Gleitmittel, Bindemittel und Sprengmittel für den Zerfall der Tablette bei Berührung mit Magensaft, vgl. z.B. S. S. Kornblum et al., J. Pharm. Sci. 62 (1973), Nr. 1, 43—49. Insbesondere im Falle der Verwendung von Lactose als Tablettierhilfsmittel gehen manche Verwender so vor, daß sie pulverförmige Lactose mit dem zu verabreichenden Wirkstoff, gegebenenfalls zusammen mit dem Sprengmittel, z.B. vernetztem Polyvinylpyrrolidon mischen, auf die Mischung eine wäßrige Lösung eines Bindemittels, z.B. Polyvinylpyrrolidon aufsprühen, das so erhaltene feuchte Pulver trocknen und nach Zugabe von Gleitmittel dann zu Tabletten verpressen.

Auch diese Methodik, die schon sehr gute Ergebnisse zeigt, befriedigt hinsichtlich aller genannten Anforderungen noch nicht vollständig. Außerdem ist die beschriebene Arbeitsweise umständlich und arbeitsintensiv und erfordert die Verwendung von organischen Lösungsmitteln bei wasserempfindlichen Wirkstoffen.

Es bestand daher die Aufgabe, ein Tablettierhilfsmittel vorzuschlagen das neben der Erfüllung der genannten Anforderungen es erlaubt, nach Mischen mit dem Wirkstoff direkt Tabletten herzustellen und das ferner erlaubt, den Anteil an Tablettierhilfsmittel zu verringern.

Diese Aufgabe wurde erfindungsgemäß mit einem Direkttablettierhilfsmittel (d.h. einem von Wirkstoffen freien Hilfsmittel) mit einer Kornverteilung von etwa 50 bis 500 µm gelöst, das besteht in inniger Vermischung aus im wesentlichen (d.h. daß das Direkttablettiermittel nur geringe Mengen, z.B. bis zu 5 Gew.-%, gegebenenfalls weitere Stoffe enthält).

A) 88 bis 96 Gew.-%, vorzugsweise 90 bis 94 Gew.-%, eines pulverförmigen für die Herstellung von Tabletten üblichen Trägers aus Lactose oder Lactose zusammen mit höchstens gleichen Mengen anderer Trägerstoffe,

B) 2 bis 6 Gew.-%, vorzugsweise 2 bis 5 Gew.-%, eines Bindemittels ausgewählt aus Hydroxipropylmethylcellulose, Hydroxipropylcellulose, Gelatine und bevorzugt Polyvinylpyrrolidon mit einem K-Wert von 20 bis 40.

C) 2 bis 10 Gew.-%, vorzugsweise 3 bis 6 Gew.-%, eines Tablettensprengmittels, ausgewählt aus der Gruppe bestehend aus vernetzter Carboxymethylcellulose, vernetzter Carboxymethylstärke, Formaldehyd-Casein und vorzugsweise vernetztem, unlöslichem Polyvinylpyrrolidon, wobei sich alle Prozentangaben auf das Direkttablettierhilfsmittel beziehen, wie es durch Sprühtrocknung, Sprühgranulierung oder Feuchtgranulierung erhältlich ist.

Nach einer bevorzugten Ausführungsform der Erfindung wird allein Lactose, z.B. Lactose EP (EP=Europ. Pharmacopoe) Monohydrat mit einer Korngröße kleiner 600 µ, als Trägermaterial verwendet. Man kann jedoch auch z.B. Mischungen aus Lactose und Mannit oder aus Lactose und Calciumphosphat verwenden.

Als Bindemittel B) kommt Hydroxipropylmethylcellulose, wie sie unter dem Handelsnamen Pharmacoat® der Firma Shinetsu, Japan, Typ A, USP XXI oder Methocel® der Firma Dow Chemical, Typ B erhältlich sich, Hydroxipropylcellulose z.B. Klucel® LF der Firma Hercules, U.S.A., Gelatine NF XVI und vorzugsweise Polyvinylpyrrolidon vom K-Wert 20 bis 40, vorzugsweise K-Wert 28 bis 32, in Betracht, letzteres wird z.B. in R. Vieweg, M. Reiher, & H. Scheurlen, Kunststoff-Handbuch, 1971, Band 11, S. 558, Munich: Hanser oder Ullmann, 4. Auflage, Band 19, S. 385—386 beschrieben. Zur Definition des K-Wertes wird auf die Povidone-Monographie USP XXI, 1985 verwiesen. Deshalb wird hiermit auf diese Literaturstellen Bezug genommen.

Das Tablettensprengmittel C) ist ausgewählt aus vernetzter Carboxylmethylstärke-Natrium, erhältlich als Primojel® der Firma Scholten, NL oder Explotab® der Firma Mendell, U.S.A. (vgl. a. R. F. Schangraw, Drug, Gosm, Ind. 122 No. 68 und 123 No. 1, 34 (1978)), vernetzter Carboxymethylcellulose z.B. Carboxymethylcellulose Natrium USP XXI (Croscarmellose Sodium) erhältlich als Nymzel® ZSB 16 der Firma Nyma, Holland oder Carboxy-AC-DI-SOL® NF XVI der Firma FMC, U.S.A., Formaldehyd-Casein z.B. erhältlich als Esma-Spreng der Firma Edelfettewerke Schlüter, Bundesrepublik Deutschland (vgl. a. B. Selmeczi, M. Liptate (Sci. Pharm. 37, 284 (1969) C.A. 72, Nr. 16-82941) und vorzugsweise vernetztem, unlöslichem Polyvinylpyrrolidon. Die Eigenschaften und die Herstellung von vernetztem, unlöslichem Polyvinylpyrrolidon, das heißt einem nicht mehr wasserlöslichen, sondern nur noch wasserquellbaren Produkt, auch Crospovidone genannt, sind in der DE—OS 22 55 263 der BASF Aktiengesellschaft (1974); bei H. F. Kauffmann & J. W. Breitenbach, Angew. Makromol. Chem., 1974, 45 167—75; J. W. Breitenbach, Proceedings of 1967 IUPAC Symposium on Macromolecular Chemistry in Budapest, pp. 529-44, und J. W. Breitenbach, H. F. Kauffmann & G. Zwilling, Makromol. Chem., 1976, *177*, 2787—92, eingehend beschrieben, so daß nur auf diese Literaturstellen Bezug genommen wird.

Bezüglich der physikalisch-chemischen Eigenschaften wird auf USP-Monographie verwiesen. Wesentliche Kriterien sind die Hydratationskapazität von 4—7 (S. Kornblum & S. Stoopak, J. Pharm., Sci., 1973, *62* S. 43—49) und folgende Kornverteilung:

max.            1% >250 μm

mind.           30% > 50 μm.

Die Herstellung der erfindungsgemäßen innigen Mischungen aus den Komponenten A), B) und C) erfolgt nach an sich bekannten Methoden, z.B. durch Sprühgranulierung, Feuchtgranulierung oder vorzugsweise durch Sprühtrocknung.

Im Falle der Sprühgranulierung geht man z.B. so vor, daß man eine Mischung aus Lactose und der Komponente C) vorlegt und im Wirbelbett bei leicht erhöhter Temperatur, z.B. 40 bis 60°C, mit einer Lösung der Komponente B) besprüht und ein getrocknetes Produkt gewinnt.

Zur Feuchtgranulierung mischt man beispielsweise die pulverförmige Lactose mit der Komponente C) in einem geeigneten Mischer vor, gießt die Lösung der Komponente B) unter weiterem Rühren zu und trocknet das Feuchtgut, nachdem man es durch ein Sieb passiert hat.

Die Sprühtrocknung wird in der Regel so ausgeführt, daß man eine wäßrige Suspension mit gelösten Anteilen aus pulverförmiger Lactose, der Komponente B und der Komponente C in einer geeigneten Sprühvorrichtung im Gegen- oder Gleichstrom zur Trockenluft bei erhöhten Temperaturen, z.B. bei bis zu 120°C Eintrittstemperatur der Trockenluft, versprüht.

Mit den genannten Verfahren, insbesondere der Sprühtrocknung, und ausgehend von feingepulverter Lactose, stellt man zweckmäßig ein Pulver mit einer engen Konverteilung von z.B. ca. 50 bis 500 μm und mit 60 bis 70% Anteilen zwischen 100 und 250 μm her.

Die erhaltenen erfindungsgemäßen Mischungen haben ausgezeichnete Tablettiereigenschaften und zeichnen sich gegenüber bekannten Direkttablettierhilfsstoffen insbesondere durch folgende Vorteile aus: Gute Fließfähigkeit, gute Komprimierbarkeit bei niedrigem Druck, ausgezeichnete Zerfallseigenschaften bei hoher Härte und geringem Abrieb der Tabletten.

Gegenüber der bekannten Technik der Mischung von Lactose mit einem Wirkstoff und vernetztem, unlöslichem Polyvinylpyrrolidon, Aufsprühen von Polyvinylpyrrolidon-Lösung, Trocknen und Tablettieren, war es überraschend, daß die Vorabherstellung des erfindungsgemäßen innigen Gemisches mit nachträglicher Einmischung des Wirkstoffs und Direktverpressung außer dem Vorteil der Einsparung von Herstellungsschritten beim Tablettenhersteller Vorteile im Eigenschaftsprofil bezüglich Möglichkeit der Einsparung von Träger wegen besserer Bindewirkung, besseren Zerfall der Tablette und leichtere Verarbeitung beim Preßvorgang bewirkte.

In den folgenden Beispielen wird die Herstellung der erfindungsgemäßen Mischungen sowie die Herstellung von Tabletten beschrieben.

Beispiele

| | | | |
|---|---|---|---|
| 1) | Lactose-Monohydrat EP Korngröße <600 μ | | 100 Teile |
| | Crospovidone | | 10 Teile |
| | Polyvinylpyrrolidon K30 (Povidone) | | 3 Teile |
| | | | |
| 2) | Lactose (wie 1) | | 160 Teile |
| | Calciumhydrogenphosphat | | 30 Teile |
| | Povidone K30 | | 6 Teile |
| | Crospovidone | | 10 Teile |
| | | | |
| 3) | Lactose (wie 1) | | 90 Teile |
| | Crospovidone | | 5 Teile |
| | Povidone K30 | | 5 Teile |
| | | | |
| 4) | Lactose (wie 1) | | 93,5 Teile |
| | Crospovidone | | 3,5 Teile |
| | Povidone K30 | | 3,0 Teile |
| | | | |
| 5) | Lactose (wie 1) | | 93,5 Teile |
| | Povidone K30 | | 3,0 Teile |
| | Formaldehyd-Casein | | 3,5 Teile |

| 6) | Lactose (wie 1) | 93,5 Teile |
| | Povidone K30 | 3,0 Teile |
| | Carboxymethylcellulose-Natrium | 3,5 Teile |

| 7) | Lactose (wie 1) | 93,5 Teile |
| | Povidone K30 | 3,0 Teile |
| | Croscarmellose Sodium | 3,5 Teile |

| 8) | Lactose (wie 1) | 93,5 Teile |
| | Povidone K30 | 3,0 Teile |
| | Carboxymethylstärke-Natrium | 3,5 Teile |

| 9) | Lactose (wie 1) | 93,5 Teile |
| | Gelatine | 3,0 Teile |
| | Crospovidone | 3,5 Teile |

| 10) | Lactose (wie 1) | 93,5 Teile |
| | Hydroxypropylmethylcellulose | 3,0 Teile |
| | Crospovidone | 3,5 Teile |

| 11) | Lactose (wie 1) | 93,5 Teile |
| | Hydroxypropylmethylcellulose | 3,0 Teile |
| | Crospovidone | 3,5 Teile |

Die vorgenannten Mischungen lassen sich durch folgende Arbeitsweisen in tablettierbare Mischungen überführen.

a) Die Lactose wird zusammen mit dem Bindemittel, in ca. gleichen Mengen Wasser gelöst bzw. suspendiert. Zur Suspension wird das Tablettensprengmittel hinzugefügt. Die so zubereitete Suspension gibt man durch eine Korundscheibenmühle und versprüht sie anschließend in der Wirbelschicht.

Sprühbedigungen:

| Zuluft | 40—80°C |
| Düse | 0,8 mm |
| Sprühdruck | 2—3 bar. |

b) Die Lactose wird mit dem Tablettensprengmittel als trockene Mischung im Wirbelschichtgranulator vorgelegt und in der Wirbelschicht mit dem Bindemittel, gelöst in der 10-fachen Menge Wasser, Sprühgranuliert.

c) Die Lactose wird mit dem Tablettensprengmittel gemischt (z.B. in einem Diosua-Mischer); dazu wird eine Lösung des Bindemittels in der 3-fachen Menge Wasser gegossen, das Gemisch 5 Minuten innig durchgemischt, die erhaltene Mischung durch ein 0,8 mm Sieb passiert, bei 40°C getrocknet und danach erneut durch ein 0,5 mm Sieb gesiebt.

Mit den genannten Methoden erhält man Direkttablettierhilfsmittel mit einer Fließfähigkeit im Fordbecher bei Öffnung 5 von weniger als 50 Sekunden und folgender Kornverteilung:

| max. | 5% <50 μ |
| max. | 70% <200 μ |
| mind. | 95% <400 μ |
| max. | 1% >500 μ. |

Tablettierungsbeispiele

| A) | Mischung 4 nach Methode b): | 99,5 Teile |
| | Gleitmittel | 0,5 Teile |

Die Komponenten werden 5 Minuten gemischt und dann auf einen Rundläufer zu Tabletten mit 500 mg Gewicht,

12 mm Durchmesser
biplan bei mittlerem Preßdruck

verarbeitet. Man erhält Tabletten mit einer Härte von 100 N und einer Zerfallsgeschwindigkeit von 2—3 min.

4

Fürht man diese Probe mit den Beispielen 5 bis 11 durch, erhält man folgende Ergebnisse:

| Mischung des beispiels | Härte | Zerfallsgeschwindigkeit |
|---|---|---|
| 5 | 65 N | 4 Minuten |
| 6 | 67 N | 3 Minuten |
| 7 | 54 N | 2—3 Minuten |
| 8 | 57 N | 3—4 Minuten |
| 9 | 58 N | 3—4 Minuten |
| 10 | 36 N | 4 Minuten |
| 11 | 44 N | 15 Minuten |

| | | |
|---|---|---|
| B) Paracetamol | 30 Teile | |
| Mischung 4, Methode b) | 70 Teile | |
| Magnesiumstearat | 0,5 Teile | |

Verarbeitung wie in Beispiel A angegeben:

| | |
|---|---|
| Härte | 70 N |
| Zerfall | 1 min. |

| | | |
|---|---|---|
| C) Phenacetin | 30 Teile | |
| Mischung 4, Methode b) | 70 Teile | |
| Magnesiumstearat | 0,5 Teile | |

Verarbeitung wie in Beispiel A angegeben:

| | |
|---|---|
| Härte | 110 N |
| Zerfall | 5 min. |

| | | |
|---|---|---|
| D) Mischung 4, Methode b) | 100 Teile | |
| Ascorbinsäure Pulver | 50 Teile | |
| Magnesiumstearat | 1 Teil | |

Verarbeitung wie in Beispiel A angegeben:

| | |
|---|---|
| Härte | 80 N |
| Zerfall | 2 min. |

**Patentansprüche**

1. Direkttablettierhilfsmittel mit einer Kornverteilung von etwa 50 bis 500 µm (max. 5% <50 µm, max. 1% >500 µm), bestehend im wesentlichen aus einer innigen Mischung von

A) 88 bis 96 Gew.-% eines pulverförmigen, für die Herstellung von Tabletten üblichen Trägers aus Lactose oder Lactose zusammen mit höchstens gleichen Mengen anderer Trägerstoffe,

B) 2 bis 6 Gew.-% eines Bindemittels, ausgewählt aus der Gruppe bestehend aus Polyvinylpyrrolidon mit einem K-Wert von 20 bis 40, Hydroxipropylmethylcellulose, Hydroxipropylcellulose und Gelatine,

C) 2 bis 10 Gew.-% eines Tablettensprengmittels, ausgewählt aus der Gruppe bestehend aus vernetztem unlöslichem Polyvinylpyrrolidon, vernetzter Carboxymethylcellulose, vernetzter Carboxymethylstärke und Formaldehyd-Casein, wobei sich alle Prozentangaben auf das Direkttablettierhilfsmittel beziehen, wie es durch Sprühtrocknung, Sprühgranulierung oder Feuchtgranulierung erhältlich ist.

2. Direkttablettierhilfsmittel nach Anspruch 1, dadurch gekennzeichnet, daß das Bindemittel B Polyvinylpyrrolidon mit einem K-Wert von 20 bis 40 ist.

3. Direkttablettierhilfsmittel nach Anspruch 1 oder 2, dadurch gekennzeichnet, daß das Tablettensprengmittel C vernetztes unlösliches Polyvinylpyrrolidon ist.

4. Direkttablettierhilfsmittel nach Anspruch 2 und 3, dadurch gekennzeichnet, daß es

B) 2 bis 5% Polyvinylpyrrolidon mit einem K-Wert von 20 bis 40 und

C) 3 bis 6% vernetztes unlösliches Polyvinylpyrrolidon enthält.

**Revendications**

1. agent auxiliaire pour le pastillage direct, d'une grosseur des grains comprise entre environ 50 et 500 µm (au maximum 5% <50 µm, au maximum 1% >500 µm, composé essentiellement d'un mélange intime de

A) 88 à 96% en poids d'un support pulvérulent usuel pour la confection de comprimés, en lactose ou lactose additionné de quantités au maximum égales d'autres supports,

B) 2 à 6% en poids d'un liant choisi dans le groupe composé de polyvinylpyrrolidone d'un indice K de 20 à 40, hydroxypropylméthylcellulose, hydroxypropylcellulose et gélatine,

C) 2 à 10% en poids d'un désintégrant de comprimés, choisi dans le groupe composé de polyvinylpyrrolidone insoluble réticulée, carboxyméthylcellulose réticulée, amidon de carboxyméthyle et formaldéhyde-caséine, tous les pourcentages se rapportant à l'agent auxiliaire pour le pastillage direct tel qu'il peut être obtenu par sécharge par atomisation, granulation par atomisation ou granulation humide.

2. Agent auxiliaire pour le pastillage direct, selon la revendication 1, caractérisé par le fait que le liant B est de la polyvinylpyrrolidone d'un indice K de 20 à 40.

3. Agent auxiliaire pour le pastillage direct, selon l'une des revendications 1 ou 2, caractérisé par le fait que le désintégrant C est de la polyvinylpyrrolidone insoluble réticulée.

4. Agent auxiliaire pour le pastillage direct, selon les revendications 2 et 3, caractérisé par le fait qu'il contient.

B) 2 à 5% de polyvinylpyrrolidone d'un indice K de 20 à 40 et

C) 3 à 6% de polyvinylpyrrolidone insoluble réticulée.

## Claims

1. A direct tableting auxiliary having a particle size distribution of from about 50 to 500 µm (no more than 5% <50 µm and no more than 1% >500 µm) and essentially consisting of an intimate mixture of

A) from 88 to 96% by weight of a pulverulent carrier conventionally used for the preparation of tablets and consisting of lactose, or lactose together with not more than the same amount of another carrier,

B) from 2 to 6% by weight of a binder selected from the group consisting of polyvinylpyrrolidone having a K value of from 20 to 40, hydroxypropylmethylcellulose, hydroxypropylcellulose and gelatine, and

C) from 2 to 10% by weight of a tablet disintegrating agent selected from the group consisting of cross-linked, insoluble polyvinylpyrrolidone, crosslinked carboxymethylcellulose, crosslinked carboxymethyl starch and formaldehyde casein, all percentages being based on the direct tableting auxiliary as obtainable by spray drying, spray granulation or wet granulation.

2. A direct tableting auxiliary as claimed in Claim 1, wherein the binder B is polyvinylpyrrolidone having a K value of from 20 to 40.

3. A direct tableting auxiliary as claimed in Claim 1 or 2, wherein the tablet disintegrating agent C is crosslinked insoluble polyvinylpyrrolidone.

4. A direct tableting auxiliary as claimed in Claims 2 and 3, which contains B) from 2 to 5% of polyvinylpyrrolidone having a K value of from 20 to 40 and C) from 3 to 6% of crosslinked insoluble polyvinylpyrrolidone.